# EUROPÄISCHE PATENTSCHRIFT

(11) **EP 0 374 615 B1**
(45) Veröffentlichungstag und Bekanntmachung des Hinweises auf die Patenterteilung: **28.08.1996**
(21) Anmeldenummer: 89122627.6
(22) Anmeldetag: 08.12.1989
(51) Int. Cl.: C07F 13/00, C07F 15/06, C07F 15/00, C07F 15/04, C07F 15/02, C07F 7/00, C07F 9/00, C07F 11/00, C07F 19/00, B01D 61/02, B01D 61/24

(54) **Verfahren zur Abtrennung von metallorganischen Verbindungen und/oder Metallcarbonylen aus ihren Lösungen in organischen Medien**
Process for the separation of organometallic compounds and/or metal carbonyls from their solution in organic media
Procédé de séparation de composés organométalliques et/ou métaux-carbonylés de leurs solutions en milieu organique

(30) Priorität: 20.12.1988 DE 3842819
(43) Veröffentlichungstag der Anmeldung: 27.06.1990
(73) Patentinhaber: HOECHST AKTIENGESELLSCHAFT, 65926 Frankfurt am Main (DE)
(72) Erfinder: Bahrmann, Helmut, Dr. Dipl.-Chem., D-4236 Hamminkeln-Brünen (DE); Haubs, Michael, Dr. Dipl.-Chem., D-6550 Bad Kreuznach (DE); Kreuder, Willi, Dr. Dipl.-Chem., D-6500 Mainz (DE); Müller, Thomas, Dipl.-Ing., D-4220 Dinslaken (DE)

(56) Entgegenhaltungen:
- EP-A- 0 325 962
- DE-A- 2 336 763
- GB-A- 1 266 180
- US-A- 3 966 595

## Beschreibung

Die Erfindung betrifft ein Verfahren zur Abtrennung von metallorganischen Verbindungen und/oder Metallcarbonylen, beide Verbindungsklassen werden im folgenden auch kurz als Metallverbindungen bezeichnet, die in organischen Medien gelöst sind, durch Membranfiltration.

Organische Verbindungen und Carbonylverbindungen der Übergangsmetalle, insbesondere Verbindungen, die als Zentralatom ein Metall der Platingruppe enthalten, finden in zunehmendem Umfang Verwendung als Katalysatoren in industriell ausgeübten chemischen Prozessen. Wesentlichen Einfluß auf die Wirtschaftlichkeit dieser Verfahren hat die Rückgewinnung des Katalysators. Sie soll möglichst verlustfrei erfolgen und geringen Aufwand erfordern.

Es hat daher nicht an Versuchen gefehlt Techniken zu entwickeln, die diesen Forderungen genügen. Dabei verfolgt man in der Hauptsache zwei Arbeitsrichtungen. Die eine begnügt sich damit, das Katalysatormetall wieder zu erhalten, nimmt also den Abbau der katalytisch wirksamen Verbindung in Kauf. Thermische Spaltung, Reduktion und Oxidation und Fällungsvorgänge dominieren diese Verfahren. Die andere Arbeitsrichtung strebt an, die katalytisch aktive Metallverbindung nicht zu zerstören, sondern sie unversehrt zurückzugewinnen, so daß sie ohne weitere Aufbereitung in den Prozeß rezirkuliert werden kann. Auf diese zweite Variante der Rückgewinnung von metallorganischen Verbindungen oder Metallcarbonylen wird im folgenden näher eingegangen.

Ein möglicher Weg zur Abtrennung metallorganischer Koordinationskomplexe aus organischen Flüssigkeiten besteht in der Anwendung selektiver Trennmembranen. So wird in der DE-OS 19 12 380 ein Verfahren beschrieben, bei dem man das Gemisch des Komplexes mit einer oder mehreren organischen Komponenten mit einer Seite einer Cellulosemembran unter Druck in Berührung bringt. Die Verhältnisse von Molekülgröße und -form des Komplexes zu Molekülgröße und -form der organischen Komponenten sind derart, daß das durch die Membran hindurchtretende Material einen verringerten Komplexgehalt hat.

Auch die DE-OS 19 53 641 beschreibt ein Verfahren zur Abtrennung metallorganischer Verbindungen aus einer Lösung der Verbindungen in einem organischen Lösungsmittel unter Verwendung einer Membran. Diese Arbeitsweise ist dadurch gekennzeichnet, daß man eine Silikonkautschukmembran einsetzt.

Weiterhin entfernt man nach einem in der GB-PS 12 66 180 beschriebenen Verfahren metallorganische Verbindungen aus ihren Lösungen in organischen Lösungsmitteln mit Hilfe einer Polyamidmembran.

Schließlich beschreibt die DE-OS 24 14 306 die Trennung von metallorganischen Verbindungen aus organischen Lösungen mit einer Polyacrylnitril-Membran.

Die vorstehend aufgeführten Trennverfahren haben jedoch den Nachteil, daß die Membranen in den zum Teil aggressiven organischen Lösungsmitteln nicht stabil sind, insbesondere bei Druck- und Temperaturbelastung quellen und dadurch ihre vorteilhaften Eigenschaften verlieren. Aus diesem Grund hat sich in der industriellen Praxis für solche Trennaufgaben kein Membranverfahren durchsetzen können.

Aus US-A-3,966,595 ist es ferner bekannt, Membranen aus aromatischen Polyamiden einzusetzen, um gezielt durch Umkehr-Osmose neutrale Liganden aus Komplexverbindungen von Metallen der Gruppe VIII des Periodensystems der Elemente zu entfernen, mit dem Ziel, ligandenärmere Komplexverbindungen zu erhalten. Die Möglichkeit einer Abtrennung der gesamten Komplexverbindung in unveränderter Form aus ihrer Lösung unter Einsatz von Polyaramidmembranen wird nicht beschrieben.

Aus DE-OS-23 36 763 ist bekannt, eine semipermeable Membran eines aromatischen Polyamids aus m-Phenylendiamin und Isophthalsäure zur Trennung einer organischen Lösung zu verwenden, die organische Metallkomplexe bestimmter Metalle und mindestens ein organisches Kohlenwasserstoffderivat mit mindestens einer Nitrilgruppe und bis zu 12 Kohlenstoffatomen umfaßt.

Es bestand die Aufgabe, ein Verfahren zu entwickeln, mit dem es gelingt, metallorganische Verbindungen und/oder Metallcarbonyle in unveränderter Form aus organischen Medien mit Hilfe solcher Membranen abzutrennen, die neben den erforderlichen Trenneigenschaften unter den gewählten Arbeitsbedingungen auch eine hohe Stabilität besitzen und eine einfache und effektive Abtrennung der metallorganischen Verbindungen bzw. der Metallcarbonyle aus organischen Medien ermöglichen.

Die Erfindung besteht somit in einem Verfahren zur Abtrennung und Rückgewinnung von metallorganischen Verbindungen und/oder Metallcarbonylen in unveränderter Form aus ihren Lösungen in organischen Medien. Es ist dadurch gekennzeichnet, daß man die Lösungen nach der Methode der Druckfiltration oder der Dialyse mit einer semipermeablen Membran aus einem aromatischen Polyamid (Polyaramid) in Kontakt bringt, wobei das aromatische Polyamid aus Terephthalsäure, p-Phenylendiamin, 1,4-Bis(4-amino-phenoxy)benzol und 3,3'-Dimethylbenzidin als Monomerkomponenten aufgebaut ist.

Als treibende Kraft für den Trennvorgang kann hierbei sowohl eine Druckdifferenz (Druckfiltration) als auch eine Konzentrationsdifferenz (Dialyse) dienen. Überraschenderweise gelingt es nach dem neuen Prozeß, metallorganische Verbindungen und/oder Metallcarbonyle unverändert, d. h. ohne daß sie zersetzt werden oder eine andere Umwandlung erfahren, nahezu verlustfrei wiederzugewinnen. Von besonderer Bedeutung ist in diesem Zusammenhang, daß die Membran weder durch Druck- noch durch Temperatureinwirkung in den organischen Medien ihre vorteilhaften Trenneigenschaften einbüßt.

Unter metallorganischen Verbindungen im Sinne der vorliegenden Erfindung versteht man Verbindungen, in denen Kohlenstoffatome organischer Gruppen an Metallatome gebunden sind. Zu den Metallen zählen auch die sogenannten Halbmetalle, wie Bor und Silicum und auch Phosphor. Als metallorganisch werden erfindungsgemäß auch solche, in einem organischen Lösungsmittel lösliche Verbindungen bezeichnet, in denen die Bindung zwischen Metall und Kohlenstoff über Stickstoff, Sauerstoff oder Schwefel erfolgt. Beispiele für diese Verbindungen sind Acetylacetonate und Dimethylglyoxime.

Die metallorganischen Verbindungen, die neben Kohlenstoff auch Stickstoff und Sauerstoff enthalten können, leiten sich vorzugsweise von den Elementen der Gruppen IVA, VA, VIA, VIIA, VIIIA und IB des Periodensystems der Elemente ab. Besondere Bedeutung haben metallorganische Verbindungen der Elemente Mangan, Kobalt, Nickel, Palladium, Platin, Iridium und Rhodium.

Der Begriff Metallcarbonyle ist nicht auf Verbindungen beschränkt, die allein aus Metall und CO bestehen, sondern erstreckt sich auch auf solche Verbindungen, die noch weitere Liganden wie Wasserstoff, Olefine, Phosphane, Acetat, Benzonitril enthalten. Als Carbonyle kommen solche von Metallen der Gruppe VIA, VIIA und VIIIA des Periodensystems in Betracht, insbesondere Carbonyle des Eisens, Kobalts, Nickels, Rutheniums, Rhodiums und Iridiums.

Die erfindungsgemäß eingesetzten Membranen bestehen aus einem aromatischen Polyamid, auch Polyaramid genannt, das aus den Monomerkomponenten Terephthalsäure, p-Phenylendiamin, 1,4-Bis(4-amino-phenoxy)benzol und 3,3'-Dimethylbenzidin aufgebaut ist und durch Polykondensation in einem dipolar aprotischen Lösungsmittel erhalten wird.

In den Polymeren können die Amine statistisch verteilt sein. Die Polyaramide können aber auch die Struktur von Blockcopolymerisaten besitzen.

Das mittlere Molekulargewicht der Polyaramide kann sich über einen weiten Bereich erstrecken. Üblicherweise beträgt es 5.000 bis 200.000. Bevorzugt werden Polyaramide mit einer Molmasse von 10.000 bis 50.000.

Zur Herstellung der erfindungsgemäßen Membranen hat sich ein Verfahren bewährt, das in der deutschen Patentanmeldung P 38 02 030 beschrieben ist. Die hier offenbarten Membranen bestehen aus einem Copolyamid, das aus drei unterschiedlichen Diaminen und einer Dicarbonsäure aufgebaut ist. Eine Lösung dieses Copolyamids in einem aprotischen, polaren Lösungsmittel von Amidtyp, z. B. N-Methyl-2-pyrollidon, wird auf eine plane Unterlage als flüssige Schicht ausgebreitet. Diese flüssige Schicht trägt man in die Fällflüssigkeit, insbesondere Wasser, ein, die mit dem Lösungsmittel der Lösung mischbar ist, das Polymerisat jedoch als Membran abscheidet. Man läßt die Fällflüssigkeit solange auf die ausgefällte Membran einwirken, bis das Lösungsmittel vollständig durch die Fällflüssigkeit ersetzt ist. Sofern erforderlich, kann die Membran noch einer Wärmebehandlung unterzogen werden. Anschließend wird die Membran, gegebenenfalls nach vorheriger Behandlung mit Glycerin, getrocknet.

Die nach dem vorstehend beschriebenen Verfahren hergestellten Membranen sind integral asymmetrisch, sie sind dem Fachmann prinzipiell bekannt. Die Membranen besitzen eine sehr dünne, trennaktive Schicht, deren Dicke 0,05 bis 5 µ beträgt und eine poröse Stützstruktur. Die Dicke der aus trennaktiver Schicht und Stützstruktur bestehenden Membran kann 10 bis 400 µ betragen, sie liegt vorzugsweise im Bereich von 50 bis 200 µ.

Die Form der Membran kann beliebig gewählt werden. Sie kann als Scheibe und insbesondere als Hohlfaser oder Kapillare ausgebildet sein, aber auch jede andere, für die vorgesehene Verwendung geeignete Gestalt haben. Maßgebend ist die Erzielung einer möglichst hohen Stabilität und überdies einer größtmöglichen Oberfläche je Volumeneinheit, um einen zufriedenstellenden Durchsatz zu erreichen.

Es empfiehlt sich, die Membran vor dem Einsatz vorzubehandeln. Im einfachsten Fall taucht man sie in die zu trennende Lösung. Aber auch andere Konditionierungsverfahren sind möglich. Wurde die Membran z.B. durch Ausfällung mit Wasser hergestellt, so ersetzt man das Wasser z.B. durch i-Propanol, indem man die Membran in i-Propanol einlegt und den Alkohol mehrfach erneuert. Danach wird das i-Propanol in gleicher Weise durch das organische Medium ersetzt, in dem die abzutrennenden Metallverbindungen gelöst sind. Art und Methode der Konditionierung der Membran bestimmen die beim erfindungsgemäßen Verfahren einzuhaltenden Arbeitsbedingungen.

Die maßgebenden Variablen, mit denen der Trennvorgang beeinflußt werden kann, sind bei gegebener gelöster Metallverbindung der angewandte Druck, die Temperatur der Lösung, die Art des Lösungsmittels und die Konzentration der Metallverbindung in der Lösung.

Die Durchführung des Trennvorganges nach dem erfindungsgemäßen Verfahren kann entweder als Druckfiltration oder als Dialyse erfolgen. Im ersten Fall muß zwischen der Retentatseite und der Permeatseite der Membran ein Druck aufrechterhalten werden, der größer ist, als der osmotische Druck des Systems, d.h. der Lösungen unterschiedlicher Konzentration zu beiden Seiten der Membran. Zweckmäßig beträgt die Druckdifferenz an der Membran 0,1 bis 15 MPa, vorzugsweise 0,1 bis 10 MPa und insbesondere 0,2 bis 2 MPa. Im zweiten Fall läßt man eine Spüllösung an der Permeatseite der Membran im Gegenstrom vorbeiströmen. Dieses als Dialyse bezeichnete Verfahren ist besonders vorteilhaft in einem Hohlfaser- bzw. Kapillarmodul durchzuführen. Als Spüllösungen eignen sich z.B. organische Lösungsmittel. Die Arbeitstemperaturen bei beiden Verfahrensvarianten betragen 0 bis 200°C und insbesondere 40 bis 130°C.

Die Konzentrationen der abzutrennenden Metallverbindungen in den Einsatzlösungen können sich über einen weiten Bereich erstrecken. Das erfindungsgemäße Verfahren erlaubt es mit Erfolg, gelöste Metallverbindungen abzutrennen, deren Konzentration wenige ppm beträgt, wie auch Metallverbindungen, deren Konzentration im Prozentbereich liegt. Es hat sich jedoch als zweckmäßig erwiesen, daß die Konzentrationen der metallorganischen Verbindungen und/oder der Metallcarbonyle im organischen Medium 20 Gew.-% nicht übersteigen. Bei technischen Verfahren sind Einsatzlösungen von Bedeutung, die 2 bis 400 Gew.-ppm der Metallverbindungen enthalten. Sie werden mit besonderem Erfolg eingesetzt.

Die lineare Strömungsgeschwindigkeit über der Membran liegt im Bereich von 0,1 bis 10 m/sec, vorzugsweise 0,5 bis 2,5 m/sec.

Der Trenneffekt beruht wahrscheinlich darauf, daß die kleinen Komponenten der eingesetzten Lösung, die je nach Herkunft z.B. nicht umgesetzte Ausgangsstoffe, Reaktionsprodukte und gegebenenfalls ein Lösungsmittl bzw. Lösungsmittelgemisch als Reaktionsmedium enthält, leichter durch die trennaktive Schicht der Membran hindurchtreten können als die Metallverbindung. Dementsprechend werden die besten Trennergebnisse erreicht, je größer das Volumen der Metallverbindung und je ausgeprägter der Größenunterschied zwischen Metallverbindung und den übrigen Bestandteilen der Lösung ist. Zweckmäßig hat die Metallverbindung einer wenigstens 50 % größeren Querschnitt als die größte organische Komponente. In erster Näherung kann man statt der Molekülgröße das Molekulargewicht der Komponenten zur Abschätzung der Güte der Trennung heranziehen. Es ist vorteilhaft, wenn der Unterschied im Molekulargewicht zwischen der Metallverbindung und den organischen Bestandteilen des Gemischs möglichst groß ist.

Das erfindungsgemäße Verfahren kann absatzweise oder kontinuierlich, in einer oder in mehreren Stufen durchgeführt werden. Im allgemeinen wird man die Membran außerhalb der Reaktionszone anordnen, um Reaktionsbedingungen und Trennbedingungen, z.B. Druck und Temperatur, unabhängig voneinander optimieren zu können.

Bei der einstufigen Variante wird die Einsatzlösung unter Druck der Membran zugeführt. Im einfachsten Fall zieht man das Permeat ab und entnimmt der Trennvorrichtung nach Erreichen der gewünschten Konzentration die angereicherte Lösung. Zur Erhöhung der Trennleistung läßt sich diese Arbeitsweise auch kontinuierlich betreiben. Dann strömt die Einsatzlösung längs der Membran, wird aufkonzentriert und ebenso wie das Permeat kontinuierlich abgezogen.

Die mehrstufige Trennung wird entweder mit parallel oder mit in Reihe geschalteten Trennstufen durchgeführt. Die Reihenschaltung, bei der in jeder Stufe das Permeat abgetrennt und die aufkonzentrierte Lösung der nächstfolgenden Trennstufe zugeleitet wird, erlaubt eine besonders effektive Nutzung des vorhandenen Systemdruckes, d.h. des Arbeitsdruckes im vorausgegangenen Verfahrensschritt und ermöglicht die Gewinnung höher konzentrierter Lösungen. Führt man dagegen das Permeat aufeinanderfolgenden Trennstufen zu, kann man die gelösten Stoffe, abhängig von der Anzahl der Trennstufen, nahezu vollständig wiedergewinnen.

Eine weitere Erhöhung der Trennleistung der Membran bei Anwendung der vorstehend beschriebenen Verfahrensvarianten erreicht man durch Erhöhung der Überströmung der Membran mit Hilfe einer Umwälzpumpe.

Schließlich kann man durch Zugabe einer Spüllösung auf der Permeatseite der Membran im Gleich- und vorzugsweise im Gegenstrom zur Aufgabeseite die Konzentration des gelösten Stoffes im Permeat verringern und dadurch die treibende Kraft (Differenz der Konzentrationen) erhöhen (Dialyseprinzip).

Das neue Verfahren hat sich u.a. ausgezeichnet zur Abtrennung von metallorganischen Verbindungen und/oder Metallcarbonylen aus Reaktionslösungen bewährt, in denen sie z.B. als homogene Katalysatoren Anwendung gefunden haben. Lediglich als Beispiele für solche Verbindungen seien folgende, in organischen Medien lösliche Metallkomplexverbindungen und die mit ihnen katalysierten Reaktionen genannt: Ni/Al-Komplexverbindungen, z.B. i-C₄H₉AlCl₂/NiCl₂[P(C₆H₅)₃] für die Dimerisierung von Butadien zu trans-1,4-Hexadien und von Butenen zu Octenen; Co/Al-Komplexverbindungen, z.B. i-C₄H₉AlCl₂/CoCl₂[P(C₆H₅)₃]₂ für die Dimerisierung von Butadien zu cis-1,4-Hexadien; phosphitmodifizierte Ni/Al-alkyl-verbindungen für die Herstellung von Cyclooctadien aus Butadien; Pd(CH₃COO)₂/P(C₂H₅)₃-Komplexverbindungen für die Herstellung von Octadien-1,7; RuHCl[P(C₆H₅)₃]₃ oder RhCl[P(C₆H₅)₃]₃ für die homogene Hydrierung von Olefinen; RhCO[P(C₆H₅)₃]₂ für die Hydroformylierung von Formaldehyd; Ni[P(p-C₆H₄.CH₃)₃]₄/H⁺ für die Hydrocyanierung von Butadien; Ir(COD)[P(C₆H₁₁)₃]Py (COD = 1,5-cyclooctadien, Py = Pyridin) für die Hydrierung cyclischer Alkene; RuCl₂[P(C₆H₅)₃] für die Hydrierung endständiger Alkene; [Ru(BINAP)] (ClO₄)₂ (BINAP = 2,2'-bis(diphenylphosphino)-1,1'-binaphthyl) für die asymmetrische Hydrierung olefinischer Doppelbindungen; HRhCO[P(C₆H₅)₃]₃ für die Hydroformylierung alpha-olefinischer Verbindungen wie Allylalkohol; Pd[P(C₆H₅)₃]₄ für die nukleophile Alkylierung von Allylsystemen und die funktionalisierende Oligomerisation von Butadien; Rhodiumkomplexverbindungen wie HRhCO[P(C₆H₅)₃]₃ und solche, die als Liganden Triphenylphosphan oder Alkyl- oder Arylammoniumsalze sulfonierter oder carboxylierter Triarylphosphane der allgemeinen Formel 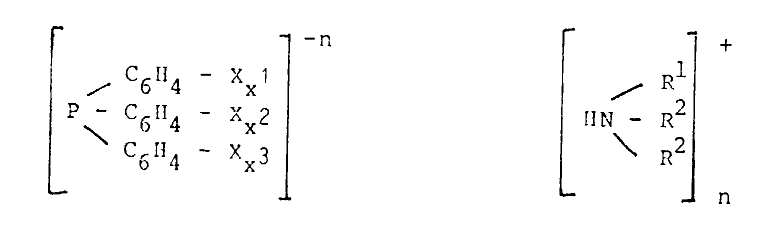 enthalten. Diese Verbindungen und ihre Verwendung werden im folgenden noch etwas näher betrachtet. Es ist aber selbstverständlich nicht beabsichtigt, den Umfang der Erfindung auf die Abtrennung dieser speziellen Verbindungsklasse zu beschränken. In der obigen allgemeinen Formel bedeuten X einen Sulfonat-(-SO₃⁻) oder Carboxylatrest (-COO⁻), x¹, x², x³ 0 oder 1, R¹, R² jeweils gleiche oder verschiedene Alkylreste mit 4 bis 12 Kohlenstoffatomen oder Aryl- oder Cycloalkylreste mit 6 bis 12 Kohlenstoffatomen und R¹ darüber hinaus noch Wasserstoff.

In solchen Katalysatorsystemen sind als Ligandanionen besonders geeignet di- und trisulfonierte bzw. -carboxylierte Triphenylphosphane. Die Verbindungen brauchen nicht in reiner Form eingesetzt zu werden, sondern können auch als Gemisch von di- und trisubstituiertem Phosphan Anwendung finden.

Die Kationen der Liganden leiten sich von sekundären bzw. tertiären Aminen ab. Bevorzugt werden Amine, die insgesamt 16 bis 36 Kohlenstoffatome enthalten. Beispiele sind Di-2-ethylhexylamin, Diisooctylamin, Diisononylamin, Tri-n-octylamin, Triisooctylamin, Triisononylamin, Triisodecylamin.

Außer den Phosphanen können die Rhodiumkomplexverbindungen noch weitere Liganden wie H, CO Amine, π-Aromaten, z.B. Cyclopentadienyl oder π-Olefine wie 1,5-Cyclooctadien enthalten.

Die Rhodiumverbindungen bilden zusammen mit dem im Überschuß vorliegenden Phosphanliganden ein Katalysatorsystem, das, homogen im organischen Reaktionsmedium gelöst, z.B. bei der Hydroformylierung olefinisch ungesättigter Verbindungen eingesetzt werden kann. Unter den Begriff olefinisch ungesättigte Verbindungen fallen geradkettige und verzweigte Olefine unabhängig von der Lage der Doppelbindung im Molekül sowie Cycloolefine wie n-Hexen-1, n-Hepten-1, n-Octen-1, n-Nonen-1, Diisobutylen, Tripropylen, Cyclohexen, Cycloocten. Zu den olefinisch ungesättigten Verbindungen zählen auch Diene wie 1,3-Butadien, 1,5-Hexadien und Dicyclopentadien sowie Verbindungen, die funktionelle Gruppen enthalten wie Acrylsäure, Acrylsäureester, Acrylnitril, Methacrylsäure, Methacrylsäureester, Vinylester, Vinylether und Acrolein.

Das bei der Hydroformylierung der olefinischen Verbindung entstehende Reaktionsgemisch enthält z.B. im wesentlichen das Reaktionsprodukt, einen Aldehyd, Nebenprodukte wie den vom Aldehyd abgeleiteten Alkohol sowie höher siedende Additions- und Kondensationsprodukte des Aldehyds. Außerdem kann noch ein Lösungsmittel, das als Reaktionsmedium dient, vorliegen.

Bevor die organischen Komponenten des Reaktionsgemisches z.B. durch Destillation isoliert werden, trennt man im allgemeinen die Rhodiumkomplexverbindung ab. Sie liegt in der Mischung üblicherweise in einer Konzentration von 1 bis 1000, insbesondere von 3 bis 400 und vorzugsweise 20 bis 200 ppm vor. Nach der neuen Arbeitsweise ist es nunmehr möglich, die Rhodiumverbindung nahezu vollständig aus dem Reaktionsprodukt abzutrennen. Von besonderer Bedeutung ist in diesem Zusammenhang, daß die Rhodiumverbindung in einer Form zurückgewonnen wird, die ihren unmittelbaren Wiedereinsatz in der Synthese erlaubt.

Nachfolgend wird die Herstellung einer Membran von der Art beschrieben, wie sie nach dem erfindungsgemäßen Verfahren eingesetzt werden kann und in den sich anschließenden Beispielen die Abtrennung von metallorganischen Verbindungen bzw. von Metallcarbonylen aus Reaktionsgemischen mit ihrer Hilfe gemäß der neuen Arbeitsweise.

### Herstellung der Membran

Das Polyaramid wird durch Kondensation von

| | |
|---|---|
| 97-99 mol% | Terephthalsäuredichlorid |
| 25 mol% | p-Phenylendiamin |
| 25 mol% | 1,4-Bis(4-aminophenoxy)benzol |
| 50 mol% | 3,3′-Dimethylbenzidin |

in N-Methylpyrrolidon als Lösungsmittel hergestellt. Terephthalsäuredichlorid setzt man in einer solchen Menge ein, daß das Polyaramid einen Staudinger-Index von 200 bis 300 ml/g hat. Die Lösungsmittelmenge wird so bemessen, daß eine etwa 7 Gew.-% Polykondensat enthaltende Lösung entsteht. Nachdem die Kondensation erfolgt ist, wird der locker an das Lösungsmittel gebundene Chlorwasserstoff durch Zugabe von 100 mol% CaO neutralisiert. Anschließend löst man in dem Reaktionsgemisch unter Rühren 5 Gew.-% (bezogen auf die Polymerlösung) wasserfreies Calciumchlorid. Die Lösung wird gelinde erwärmt, filtriert und entgast. Sie kann unmittelbar zur Membranherstellung verwendet werden.

Es ist möglich, die Membran trägerfrei oder auf einem Polyestervlies als Träger herzustellen. Im folgenden wird die Herstellung einer trägerfreien Membran beschrieben. Die leicht erwärmte Polyaramidlösung wird mit einer Rakel auf einer Glasplatte zu einem gleichmäßigen Film von etwa 150 µ ausgezogen und in ein Wasserbad von 2°C eingetaucht. Nach etwa 20 min zieht man die Membran von der Glasplatte ab und legt sie 5 min in 100°C heißes Wasser. Darauf gibt man die Membran in i-Propanol, um die Porenflüssigkeit Wasser gegen den Alkohol auszutauschen. Anschließend wird die Membran etwa 10 h in Toluol gelegt, nach dieser Behandlung ist sie zur Durchführung der Trennungen geeignet. Bei allen Operationen ist darauf zu achten, daß die Membran nicht austrocknet.

### Beispiel 1

Nachstehend wird die Abtrennung des aus einer Rhodiumkomplexverbindung und dem Triisooctylammoniumsalz von Tris(m-sulfophenyl)-phosphan bestehenden Katalysators aus dem Rohprodukt der Hydroformylierung von Dicyclopentadien (DCP) beschrieben.

Die Trennung wird mit 2.646 g Rohprodukt durchgeführt, das TCD-monoaldehyd und -dialdehyd (TCD = Tricyclodecan) im Gewichtsverhältnis 18 : 98,2, 24,8 ppm Rhodium (entsprechend 65.6 mg), 698 ppm Gesamtphosphor, d.h. P(III) und P(V) (entsprechend 1.846,9 mg) davon 17,4 mmol P(III)/kg (entsprechend 1.427,2 mg) sowie Toluol als Lösungsmittel enthält.

Das Rohprodukt wird bei 40°C und einem Druck von 0,5 MPa, einer Metallzelle, die mit einer Membran nach der vorstehenden Vorschrift hergestellten, von etwa 20 cm² Fläche versehen ist, zugeleitet. Nicht durch die Membran hindurchtretende Lösung wird mit einer Geschwindigkeit von etwa 8 l/h im Kreis geführt. Die Überströmgeschwindigkeit beträgt etwa 0,15 m/sec.

Nach Beendigung des Versuchs erhält man 2.139,4 g Permeat (81 % des Einsatzes) und 485,6 g Retentat (18,4 des Einsatzes). Im Permeat finden sich 357,3 mg Gesamtphosphor (19,3 % des Einsatzes), davon 172,4 mg als P(III)-verbindung (12,1 % des Einsatzes) und 2,29 mg Rhodium (3,5 % des Einsatzes). Im Retentat sind 77,9 % des Gesamtphosphors (bezogen auf Einsatz), und 95 % des Rhodiums (bezogen auf Einsatz) enthalten. Der Permeatfluß beträgt zu Beginn der Trennung 12 l/(m² . h), bei Beendigung des Versuchs 5 l/(m² . h).

Unter den Temperatur- und Druckbedingungen des ersten Trennschrittes werden 1.933 g des Permeats einer zweiten Membranfiltration unterworfen. Der Permeatfluß beträgt zu Beginn des Versuchs 17,5 1/(m². h), bei Beendigung 10 1/(m² . h). Die Ergebnisse der Trennung sind in der folgenden Tabelle 1 zusammengestellt.

**Tabelle 1**

| | Permeat | Retentat |
|---|---|---|
| Menge (% v.E.)* | 73,1 | 7,2 |
| P III (% v.E.) | 8,8 | 13,1 |
| Gesamt-P (% v.E.) | 5,6 | 13,2 |
| Rh (% v.E.) | 0,4 | 4,3 |

| | | |
|---|---|---|
| * v.E. = vom Einsatz | | |

Beispiel 1 zeigt, daß bei Anwendung des erfindungsgemäßen Verfahrens über 99,5 % des Rhodiums und 94,4 % der Phosphor(III)-verbindung zurückgehalten werden.

### Beispiel 2

Die vereinigten Retentate aus Beispiel 1 werden als Katalysator zur Hydroformylierung von DCP in Toluol als Lösungsmittel verwendet. Nach Beendigung der Reaktion ergibt sich aus der GC-Analyse ein Umsatz von 97,8 % und ein Verhältnis von Monoaldehyd zu Dialdehyd von 2,0 : 98,0. Der Permeatfluß beträgt bei 40°C und 0,5 MPa Druck zu Beginn 10,5 1/(m². h) und gegen Ende der Trennung 3,6 1/(m² . h). Die Rückhalteraten entsprechen denen des Beispiels 1.

Beispiel 2 zeigt, daß nach dem erfindungsgemäßen Verfahren das Katalysatorsystem und überschüssiger Ligand abgetrennt und in aktiver Form rezirkuliert werden können.

### Beispiel 3

Auch Lösungen, die Metallkomplexverbindungen in höherer Konzentration enthalten, können erfolgreich nach der neuen Arbeitsweise behandelt werden. Das Produkt der Hydroformylierung von DCP unter Verwendung des Katalysatorsystems von Beispiel 1, jedoch mit einer Rh-Konzentration von 102 ppm, wird als Ausgangsmaterial verwendet. Es enthält TCD-dialdehyd, 16,5 mmol P(III)/kg, 681 ppm Gesamtphosphor und daneben Toluol als Lösungsmittel.

Die Trennung erfolgt unter den Temperatur- und Druckbedingungen und unter Verwendung der Zelle es Beispiels 1. Im 1. Durchgang beträgt der Permeatfluß zu Beginn des Versuchs 10 l/(m² . h) und am Ende 3 l/(m² h); im 2. Durchgang, in dem das Permeat der ersten Trennung eingesetzt wird, 17 bzw. 9 l/(m² . h).

Die Ergebnisse der Trennung sind in Tabelle 2 zusammenge stellt

**Tabelle 2**

| | Permeat I / II | | Retentat I / II | |
|---|---|---|---|---|
| Menge (g) | 2.383,0 | 2.119 | 403,8 | 217 |
| P III (% v.E.) | 12,9 | 8,7 | 67,0 | 12,3 |
| P (% v.E.) | 16,8 | 6,3 | 76,5 | 12,0 |
| Rh (% v.E.) | 2,5 | 0,2 | 96,3 | 2,9 |

Beispiel 3 zeigt, daß sich auch bei höheren Rhodiumkonzentrationen der Rückhalt des Metalls und des Liganden nicht verschlechtern.

### Beispiel 4

420 g eines Produkts aus der Hydroformylierung von Hexadien-1,5 mit Rhodium und Triphenylphosphan als Katalysator werden unter Verwendung der Membranzeile des Beispiels 1 nach dem erfindungsgemäßen Verfahren getrennt. Das Oxorohprodukt enthält 333 ppm Rh und 30 % Toluol. Das Verhältnis Monoaldehyd zu Dialdehyd ist 15 zu 85. Der Permeatfluß bei 40°C beträgt 5 l/(m² . h).

Die Membranfiltration führt zu den in Tabelle 3 zusammengestellten Ergebnissen:

**Tabelle 3**

| | Permeat | Retentat |
|---|---|---|
| Menge (g) | 137 (32,4 % v.E.) | 270 (67,5 % v.E.) |
| Rh (ppm) | 192 (19 % v.E.) | 400 (81 % v.E.) |

Wie ersichtlich, werden 80 % des im Rohprodukt enthaltenen Rhodiums abgetrennt.

Die folgenden Beispiele weisen die vorteilhaften physikalischen Eigenschaften der erfindungsgemäß verwendeten Membranen nach. Es wird eine nach der vorstehenden Vorschrift hergetellte und in einer Zelle angeordnete Membran von durchschnittlich 270 µ Dicke verwendet.

Zur Trennung wird das Reaktionsgemisch aus der Hydroformylierung von DCP mit Rhodium und dem Triisooctylammoniumsalz von Tris(m-sulfophenyl)-phosphan als Katalysator eingesetzt. Die Rhodium-Konzentration im Oxorohprodukt beträgt 25,4 ppm, es enthält ferner 23,4 mmol Gesamt-P/kg, 0,013 mol P(III) und etwa 55 Toluol. Das Verhältnis von Monoaldehyd zu Dialdehyd ist 2 zu 98. Der Rh- und P-Rückhalt in den folgenden Beispielen 5 bis 10 liegt in der gleichen Größenordnung wie in den Versuchen des Beispiels 1.

### Beispiel 5

Zur Konditionierung wird die Membran nach Messung ihrer Dicke zunächst mit Aceton, dann mit Isopropanol gewaschen und isopropanolfeucht in die Apparatur eingebaut. Unmittelbar darauf wird die Apparatur mit Toluol gefüllt und das Isopropanol von der Membran durch Auswaschen verdrängt.

Anschließend wird bei 25°C und 0,3 MPa Druck der Permeatfluß gemessen. Er stabilisiert sich sehr schnell von anfangs 22 l/(m² . h) bei 18 bis 19 l/(m² . h).

### Beispiel 6

Um den Permeatfluß und die Eigenschaften der Membran gegenüber einem rhodium- und phosphanfreien, rohen TCD-dialdehyd aus der Hydroformylierung von DCP zu ermitteln, wird das Toluol durch ein, auf andere Weise von Rhodium und Phosphan befreites Produkt verdrängt. Der Permeatfluß der Membran ändert sich nicht.

Dieser Versuch zeigt, daß die Membran im Oxorohprodukt stabil bleibt, d.h. keine Quellung erfolgt.

### Beispiel 7

Bei 0,3 MPa und 25°C wird die Membran mit dem oben beschriebenen Oxorohprodukt beschickt. Über eine Stunde wird in 12 Messungen der Permeatfluß bestimmt. Es stellt sich momentan ein durchschnittlicher konstanter Permeatfluß von 5,7 l/(m² . h) ein.

### Beispiel 8

Unter einem Druck von 0,5 MPa, im übrigen aber unter den Bedingungen des Beispiels 7, wird über einen Zeitraum von 30 min in 6 Einzelmessungen der Permeatfluß bestimmt. Es wird ein durchschnittlicher Wert von 9,7 l/(m² . h) ermittelt. Beispiel 8 zeigt einen fast linearen Anstieg des Permeatflusses mit der Druckdifferenz im Bereich von 0,3 bis 0,5 MPa.

### Beispiel 9

Durch allmähliche Erhöhung der Temperatur von 25°C auf 40°C steigt der Permeatfluß kontinuierlich auf 13,6 l/(m² . h) an. Damit liegt sein Temperaturkoeffizient bei +2,5 %/°C.

### Beispiel 10

Das in den vorangegangenen Beispielen verwendete Rohprodukt aus der Hydroformylierung von DCP wird wieder durch Toluol ersetzt. Der Permeatfluß steigt spontan auf 40 l/(m² . h) bei 41°C und stabilisiert sich bei 26 l/(m² . h) bei 27°C und 0,5 MPa. Nach 1 h wird der Versuch abgebrochen, die Membran ausgebaut und eine Dickenmessung durchgeführt. Die Dicke bleibt praktisch unverändert.

Beispiel 10 zeigt, daß kein Zusetzen oder Fouling der Membran eintritt und die Membran stabil bleibt.

Die Beispiele 5 bis 10 belegen insgesamt die Leistungsfähigkeit des neuen Verfahrens.

### Beispiel 11

Die bisher verwendete Membranzelle mit einer nach dem oben beschriebenen Herstellungsverfahren erhaltenen Polyaramidmembran wird mit einem C₉-Aldehyd aus der Hochdruckhydroformylierung von Diisobutylen mit Rhodium beschickt. Der Rhodiumgehalt, das Metall liegt als Carbonylverbindung vor, im Oxorohprodukt beträgt 4,3 ppm. Das Produkt enthält keinen Phosphor. Der Permeatfluß beträgt zu Beginn des Versuches bei 40°C und 0,5 MPa 39 l/(m² . h). Er fällt beim Übergang auf das C₉-Oxorohprodukt im 1. Durchgang auf 16,8 l/(m² . h) ab und beträgt am Ende des Versuchs 11,6 l/(m² . h); die Permeatflüsse im 2. Durchgang (Einsatz des Permeats der ersten Trennung) betragen entsprechend 14,5 bzw. 10,1 l/(m² . h).

Die Ergebnisse sind in Tabelle 4 zusammengestellt:

**Tabelle 4**

| | Permeat 1 | Permeat 2 | Retentat 1 | Retentat 2 |
|---|---|---|---|---|
| Menge (g) | 625 | 517 | 132 | 104 |
| Rh (% v.E.) | 52 | 18 | 35 | 27,9 |

Nach Beendigung des Versuchs und Austausch des Oxorohprodukts gegen Toluol wird wieder ein Permeatfluß von 40 l/(m² . h) gemessen.

## Patentansprüche

1. Verfahren zur Abtrennung und Rückgewinnung von metallorganischen Verbindungen und/oder Metallcarbonylen in unveränderter Form aus ihren Lösungen in organischen Medien, dadurch gekennzeichnet, daß man die Lösungen nach der Methode der Druckfiltration oder der Dialyse mit einer semipermeablen Membran aus einem aromatischen Polyamid (Polyaramid) in Kontakt bringt, wobei das aromatische Polyamid aus Terephthalsäure, p-Phenylendiamin, 1,4-Bis(4-amino-phenoxy)benzol und 3,3'-Dimethylbenzidin als Monomerkomponenten aufgebaut ist.

2. Verfahren nach Anspruch 1, dadurch gekennzeichnet, daß das aromatische Polyamid ein statistisches oder ein Blockcopolyaramid ist.

3. Verfahren nach Anspruch 1 oder 2, dadurch gekennzeichnet, daß das Molekulargewicht, angegeben als Gewichtsmittel des Polyaramids, 5.000 bis 200.000, vorzugsweise 10.000 bis 50.000 beträgt.

4. Verfahren nach einem oder mehreren der Ansprüche 1 - 3, dadurch gekennzeichnet, daß die Membran als Kapillare oder als Hohlfaser ausgebildet ist.

5. Verfahren nach einem oder mehreren der Ansprüche 1 - 4, dadurch gekennzeichnet, daß die Stärke der trennaktiven Schicht der Membran 0.05 bis 5 µ beträgt.

6. Verfahren nach einem oder mehreren der Ansprüche 1 - 5, dadurch gekennzeichnet, daß die Druckdifferenz über die Membran 0,2 - 2,0 MPa beträgt.

7. Verfahren nach einem oder mehreren der Ansprüche 1 - 6, dadurch gekennzeichnet, daß die Abtrennung der Metallverbindungen bei Temperaturen von 0°C bis 200°C, insbesondere 40 bis 130°C erfolgt.

8. Verfahren nach einem oder mehreren der Ansprüche 1 - 7, dadurch gekennzeichnet, daß sich die metallorganischen Verbindungen von Metallen der Gruppen IVA, VA, VIA, VIIA, VIIIA oder IB des Periodensystems ableiten.

9. Verfahren nach Anspruch 8, dadurch gekennzeichnet, daß die metallorganischen Verbindungen sich von Mangan, Kobalt, Rhodium, Iridium, Nickel, Palladium oder Platin ableiten.

10. Verfahren nach einem oder mehreren der Ansprüche 1 - 7, dadurch gekennzeichnet, daß sich die Metallcarbonyle von Metallen der Gruppen VIA, VIIA oder VIIIA des Periodensystems ableiten.

11. Verfahren nach Anspruch 10, dadurch gekennzeichnet, daß sich die Metallcarbonyle von Eisen, Kobalt, Nickel, Ruthenium, Rhodium oder Iridium ableiten.

12. Verfahren nach einem oder mehreren der Ansprüche 1 - 11, dadurch gekennzeichnet, daß die Konzentration der Metallverbindungen in der Lösungen 2 bis 400 Gew.-ppm (bezogen auf die Lösung) beträgt.

## Claims

1. A process for separating and recovering organometallic compounds and/or metal carbonyls in unchanged form from their solutions in organic media, characterised in that the solutions are brought into contact with a semipermeable membrane made of an aromatic polyamide (polyaramide) using the pressure filtration method or dialysis, the aromatic polyamide being synthesised from terephthalic acid, p-phenylene diamine, 1,4-bis(4-aminophenoxy)benzene and 3,3'-dimethylbenzidine as monomer components.

2. A process according to claim 1, characterised in that the aromatic polyamide is a statistical or a block copolyaramide.

3. A process according to claim 1 or 2, characterised in that the molecular weight, given as the average weight of the polyaramide, is 5,000 to 200,000, preferably 10,000 to 50,000.

4. A process according to one or more of claims 1 to 3, characterised in that the membrane has the form of a capillary or hollow fibre.

5. A process according to one of more of claims 1 to 4, characterised in that the thickness of the separating layer of the membrane is 0.05 to 5 µm.

6. A process according to one or more of claims 1 to 5, characterised in that the difference in pressure across the membrane is 0.2 to 2.0 MPa.

7. A process according to one or more of claims 1 to 6, characterised in that the metal compounds are separated at temperatures of 0 to 200°C, in particular 40 to 130°C.

8. A process according to one or more of claims 1 to 7, characterised in that the organometallic compounds are derived from metals of groups IVA, VA, VIA, VIIA, VIIIA or IB of the periodic table.

9. A process according to claim 8, characterised in that the organometallic compounds are derived from manganese, cobalt, rhodium, iridium, nickel, palladium or platinum.

10. A process according to one or more of claims 1 to 7, characterised in that the metal carbonyls are derived from metals of groups VIA, VIIA or VIIIA of the periodic table.

11. A process according to claim 10, characterised in that the metal carbonyls are derived from iron, cobalt, nickel, ruthenium, rhodium or iridium.

12. A process according to one or more of claims 1 to 11, characterised in that the concentration of the metal compounds in the solutions is 2 to 400 ppm by weight (related to the solution).

## Revendications

1. Procédé pour séparer et récupérer des composé organiques de métaux et/ou des métaux-carbonyles tels quels de leurs solutions dans des milieux organiques, caractérisé en ce que l'on met les solutions en contact par la technique de filtration sous pression ou la technique de dialyse avec une membrane semi-perméable consistant en un polyamide aromatique (polyaramide) qui a été préparé à partir des composants monomères : acide téréphtalique, p-phénylènediamine, 1,4-bis-(4-aminophénoxy)benzène et 3,3'-diméthylbenzidine.

2. Procédé selon la revendication 1, caractérisé en ce que le polyamide aromatique est un copolyaramide statistique ou séquencé.

3. Procédé selon la revendication 1 ou 2, caractérisé en ce que le poids moléculaire moyen, moyenne en poids, du polyaramide est de 5 000 à 200 000, de préférence de 10 000 à 50 000.

4. Procédé selon une ou plusieurs des revendications 1 à 3, caractérisé en ce que la membrane est sous forme de capillaires ou sous forme de fibres creuses.

5. Procédé selon une ou plusieurs des revendications 1 à 4, caractérisé en ce que l'épaisseur de la couche séparatrice active de la membrane est de 0,05 à 5 µm.

6. Procédé selon une ou plusieurs des revendications 1 à 5, caractérisé en ce que la différence de pression sur la membrane est de 0,2 à 2,0 MPa.

7. Procédé selon une ou plusieurs des revendications 1 à 6, caractérisé en ce que l'on sépare les composés métalliques à des températures de 0 à 200°C, plus spécialement de 40 à 130°C.

8. Procédé selon une ou plusieurs des revendications 1 à 7, caractérisé en ce que les composés organiques de métaux dérivent de métaux des groupes IVA, VA, VIA, VIIA, VIIIA ou IB de la Classification Périodique.

9. Procédé selon la revendication 8, caractérisé en ce que les composés organiques de métaux, dérivent du manganèse, du cobalt, du rhodium, de l'iridium, du nickel, du palladium ou du platine.

10. Procédé selon une ou plusieurs des revendications 1 à 7, caractérisé en ce que les métaux-carbonyles dérivent de métaux des groupes VIA, VIIA ou VIIIA de la Classification Périodique.

11. Procédé selon la revendication 10, caractérisé en ce que les métaux-carbonyles dérivent du fer, du cobalt, du nickel, du ruthénium, du rhodium ou de l'iridium.

12. Procédé selon une ou plusieurs des revendications 1 à 11, caractérisé en ce que la concentration des composés métalliques dans les solutions est de 2 à 400 ppm en poids (par rapport à la solution).
